# EUROPEAN PATENT APPLICATION

(11) **EP 1 181 899 A2**
(43) Date of publication of application: **27.02.2002**
(21) Application number: 01119674.8
(22) Date of filing: 22.08.2001
(51) Int. Cl.: A61F 2/00, A61L 31/00

(54) **Areal implant**

(30) Priority: 23.08.2000 DE 10041347
(71) Applicant: Ethicon GmbH, 22851 Norderstedt (DE)
(72) Inventor: Barault, Jean-Francois, Dr., 71100 Saint Remy (FR)
(74) Representative: UEXKÜLL & STOLBERG

(57) **Abstract**

An areal implant has a mesh-like basic structure (30) which is provided with a reinforced zone (32) in a central area. Radial reinforcing elements (34, 36) can extend from the reinforced zone (32) towards the peripheral edge (31) of the basic structure (30).

## Description

The invention relates to an areal implant which is suitable in particular for treating inguinal hernias (ruptures), including by means of laparoscopic surgery.

During the surgical treatment of a hernia, the hernia defect is bridged with the help of an areal implant. The conventional implants used consist mostly of a more or less flexible mesh into the pores of which tissue can grow. Implant meshes made from polypropylene are often used, but completely or partially resorbable implants are also used.

In order to ensure sufficient strength, conventional areal implants have a relatively high weight per surface unit. This is associated with a considerable burden to the patient by the material foreign to the body, which is not completely harmless from a medical point of view. If such an implant is relatively rigid, the patient will also find it uncomfortable. Often, such relatively heavy implants or the cicatricial tissue caused thereby also hinders access to organs located behind same in later operations.

The object of the invention is to provide an areal implant which is constructed as light as possible, but nevertheless has sufficient strength so that it can be used, e.g., in the surgical treatment of inguinal hernias.

This object is achieved by an areal implant with the features of claim 1. Advantageous designs of the invention are given in the dependent claims.

The areal implant according to the invention has a mesh-like basic structure and is provided with (at least) one reinforced zone in a central area of the basic structure. The area with a reinforced zone need not be arranged in the geometric centre of the implant, however.

The structure of the implant according to the invention makes possible a generally low weight per unit area and high flexibility, little amount of foreign material in the body of the patient and good healing behaviour. In spite of this, sufficient strength is guaranteed, as the implant is, as a rule, used in such a way that the reinforced zone is located in the operation area which is subjected to the greatest stress. For example, the reinforced zone can be placed directly above the hernia defect during a hernia operation so that penetration of the hernial sac is prevented. On the other hand, the peripheral area of the basic structure serves to safely fix the implant to resistant tissue, e.g. with the help of suture material or clips. At points where no fixing is provided, it is conceivable to minimise the weight per unit area arranging for the implant to have the largest possible pores or meshes there. In a similar way, cicatricial hernias can also be treated with the implant according to the invention. The implant is preferably so flexible that it can be inserted into the body of the patient endoscopically.

In a preferred version of the invention the strength of the reinforced zone decreases towards the peripheral area of the basic structure. For example, the reinforced zone can have a homogenous central area (i.e. a central area of uniform strength) which is surrounded by a zone of lower strength. An implant designed in this way is optimally matched to the stress to be expected in the patient's body so that no superfluous material is present, the mentioned negative effects thereby being extensively avoided.

The reinforced zone is preferably mesh-like and has a smaller pore size than the peripheral area of the basic structure. It is sufficient, for the treatment of for example an inguinal hernia, if the pore size in the peripheral area of the basic structure is of the order of 4 to 8 mm, which is sufficient to fix the implant there with the help of clips or a suture. Pores of such size offer the additional advantage that the peripheral area of the basic structure is practically transparent. On the other hand, the mesh-like reinforced zone preferably has significantly smaller pores, e.g. of the order of 1 to 2 mm, to ensure the required strength and to, e.g., largely hold back a hernial sac. It is, however, also conceivable to provide a relatively large pore size (e.g. 6 to 8 mm) in the reinforced zone, but to use stronger material there than in the peripheral area of the implant.

In preferred versions of the implant according to the invention, radial reinforcing elements extend from the reinforced zone towards the peripheral edge of the basic structure. At least one radial reinforcing element can be widened in the area of the peripheral edge of the basic structure. With such an implant, the basic structure can thus be reinforced in a spider web-like manner. In this way, a particularly high strength can be achieved while the overall weight is still small. For example, suture stitches or clips can be used to fix the implant in the area of the radial reinforcing elements, which offers a high degree of security against their being torn out. During the treatment of ruptures, the widened area of a reinforcing element can be placed over the Coopers ligament, to which the implant can be particularly securely fixed.

The basic structure is preferably weft-knitted or warp-knitted. In a preferred version, the entire implant is weft-knitted or warp-knitted as one piece. When the implant is made in one piece, the reinforced zone and optionally the radial reinforcing elements can, e.g., be warp-knitted into the basic structure or worked in in another suitable way.

In another preferred version, the reinforced zone is made in one piece in the form of a reinforcing part, and preferably weft-knitted or warp-knitted, and attached to the separately produced basic structure (which preferably is likewise weft-knitted or warp-knitted). If radial reinforcing elements are present, the reinforced zone can be made in one piece together with the radial reinforcing elements in the form of a reinforcing part (preferably weft-knitted or warp-knitted) and attached to the separately produced basic structure (which is also preferably weft-knitted or warp-knitted). The reinforcing part can be e.g. glued or sewn onto the basic structure.

The implant according to the invention can consist of non-resorbable material, resorbable material or a combination of resorbable and non-resorbable material. Monofilaments or multifilaments made from polypropylene, polyamide or polyester or combinations of these materials, for example, can be used as non-resorbable material. Suitable resorbable materials are, e.g., copolymers of glycolide and lactide (e.g. polyglactin 910, a copolymer made from 9 parts glycolide and one part lactide), poly-p-dioxanone or combinations of these.

In preferred versions, the implant has a stiffening element made from resorbable material, which can be formed, e.g., as a coating of material of the implant or as a film. Such a stiffening element has the effect that an implant which is very soft and flexible as a result of a basically low mass per unit area is somewhat heavier, but above all stiffer and more solid during the surgical operation, which makes it much easier to handle. The stiffening resorbable material is resorbed in the patient's body after the operation, so that after some time, which depends on the properties of the resorbable materials known per se, the implant has the desired low mass per unit area.

The individual parameters of the implant according to the invention, such as, e.g., the choice of material, the pore size, the type of knitting, the size or the strength of the basic structure and the reinforced zone, depend in detail on the field of application of the implant. A plurality of versions is possible. There may be cited as examples at this point a non-resorbable basic structure made from polypropylene with a pore size of approx 6 mm and a reinforced zone which is warp-knitted from a mixture of polypropylene monofilaments and resorbable threads made from polyglactin 910 with a pore size roughly four times smaller, as well as an implant with a basic structure and a reinforced zone made from polyamide which is coated with polyglactin 910. It is also conceivable to prefabricate the implant with an over-sized basic structure, so that the surgeon can cut the implant to the desired size during the operation.

The invention is explained in more detail in the following using further embodiments. The drawings show in
- Figure 1: a top view of a first version of the implant according to the invention,
- Figure 2: a top view of a second version of the implant according to the invention and
- Figure 3: a top view of a third version of the implant according to the invention.

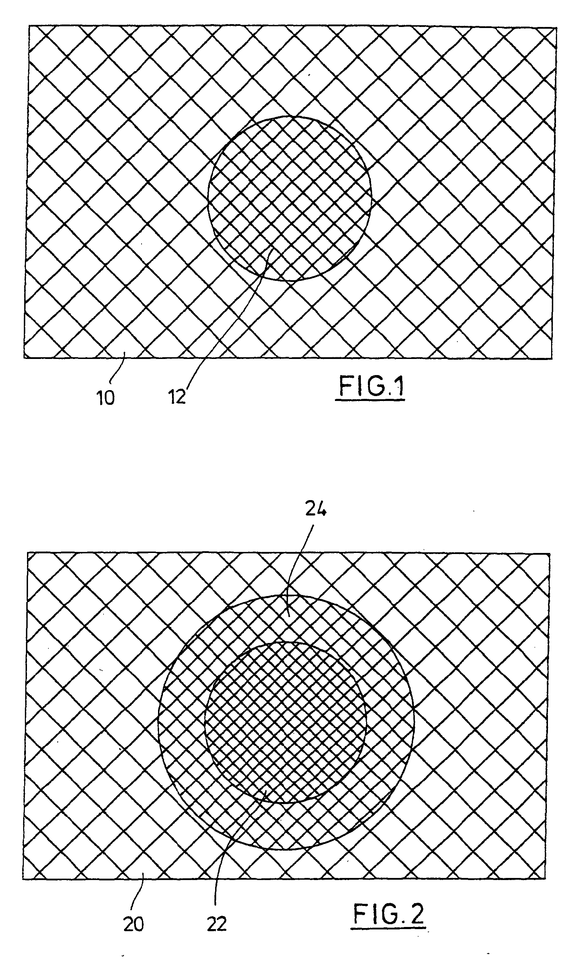

The version shown in Figure 1 of an areal implant has a mesh-like basic structure 10 which in the embodiment is warp-knitted from polypropylene multifilament yarn with a pore size of approx 4 mm. A circular reinforced zone 12 made from a warp-knitted composite material is sewn onto the middle area of the continuous basic structure 10, which composite material consists of polypropylene monofilaments (non-resorbable) and monofilaments made from the resorbable material polyglactin 910. The pore size of the reinforced zone 12 is 1 mm in the embodiment.

The reinforced zone 12 has a diameter of 5 cm in the embodiment. The basic structure 10 extends beyond the area shown in Figure 1 and in the embodiment is quadratic with a side length of 20 cm. It can be cut to a suitable size if needed before or during the operation.

The material of the basic structure 10 and the reinforced zone 12 is provided in the embodiment with a stiffening coating made from polyglactin 910.

In the version of an areal implant shown in Figure 2, a mesh-like basic structure 20 is provided in its middle area with a reinforced zone which is in two parts and has a central area 22 and a zone 24 surrounding this, the strength of which is lower than that of the central area 22. In the embodiment, the basic structure is warp-knitted from a polypropylene multifilament with a pore size of approx 5 to 6 mm and cut out in its middle area to the size of the reinforced zone 22, 24. The surrounding zone 24 is likewise warp-knitted from polypropylene multifilament, but has a pore size of only approx 3 mm. It is sewn in as a circular element into the cavity in the basic structure 20. The central area 22 is also warp-knitted from polypropylene multifilament yarn, with a pore size of approx 1 mm, and laid onto the middle area of the circular element for the surrounding zone 24 and sewn there.

In the embodiment the central area 22 has a diameter of 5 cm and the annular surrounding zone 24 has a width of 4 cm; the circular element from which the surrounding zone 24 is constructed therefore has a diameter of 13 cm. The basic structure 20, which is larger in the embodiment than is shown in Figure 2, is quadratic and has a side length of 25 cm. The representation in Figure 2 is not to scale.

In the version according to Figure 2, the strength decreases from the centre outwards in a total of three steps, due to the increasing pore size. It is therefore optimally matched, with minimal use of material, to the stresses acting on the implant.

The handling of the version according to Figure 2 is improved in the way explained above by a coating made from the resorbable material polyglactin 910.

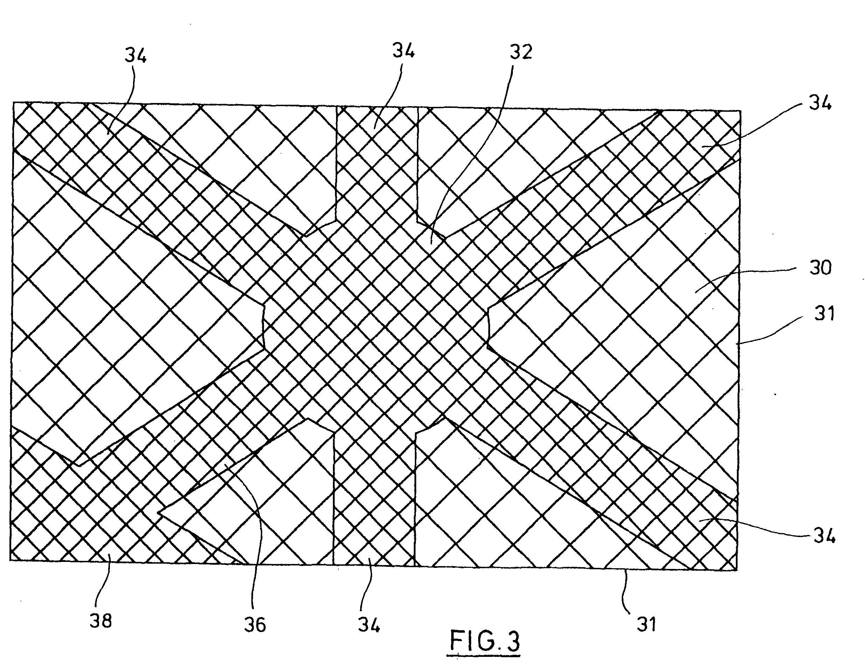

Figure 3 shows a third version of an areal implant. A basic structure 30 which extends over the entire area of the implant, with a peripheral edge 31, is warp-knitted from polypropylene multifilament yarn and has a pore size of approx 6 - 8 mm. A separately produced reinforcing part is sewn onto the basic structure 30, which consists of a reinforced zone 32, which comes to rest in the middle area of the basic structure 30, and radial reinforcing elements 34 and 36 which extend as far as the peripheral edge 31 of the basic structure 30. The radial reinforcing element 36 has a widened section 38 at its outer end in the area of a corner of the implant.

The reinforcing part is cut out from a warp-knitted product made from polypropylene multifilament yarn of a pore size of approx 2 mm and secured against unravelling at the cut edges by a heat treatment. It not only reinforces the middle area of the basic structure 30, but also offers via the radial reinforcing elements 34 and 36 tear-resistant anchoring points at which the implant can be sewn or clamped to the tissue during the surgical operation. The widened section 38 of the radial reinforcing element 36 serves for attachment to the patient' s Cooper' s ligament.

The polypropylene multifilament yarn used is coated with the resorbable material polyglactin 910 to improve the handling properties of the implant.

## Claims

1. Areal implant with a mesh-like basic structure (10; 20; 30) and a reinforced zone (12; 22, 24; 32) in a central area of the basic structure (10; 20; 30).

2. Implant according to claim 1, **characterized in that** the strength of the reinforced zone (22, 24) decreases towards the peripheral area of the basic structure (20).

3. Implant according to claim 2, **characterized in that** the reinforced zone (22, 24) has a homogenous central area (22) and a zone (24) of lower strength surrounding the homogenous central area (22).

4. Implant according to one of claims 1 to 3, **characterized in that** the reinforced zone (12; 22, 24; 32) is mesh-like and has a smaller pore size than the peripheral area of the basic structure (10; 20; 30).

5. Implant according to one of claims 1 to 4, **characterized in that** radial reinforcing elements (34, 36) extend from the reinforced zone (32) towards the peripheral edge (31) of the basic structure (30).

6. Implant according to claim 5, **characterized in that** at least one radial reinforcing element (36) is widened in the area of the peripheral edge (31) of the basic structure (30).

7. Implant according to one of claims 1 to 6, **characterized in that** the basic structure (10; 20; 30) is weft-knitted or warp-knitted.

8. Implant according to claim 7, **characterized in that** the implant is weft-knitted or warp-knitted in one piece.

9. Implant according to one of claims 1 to 7, **characterized in that** the reinforced zone (12) is made in one piece in the form of a reinforcing part, preferably weft-knitted or warp-knitted, and is attached to the separately produced basic structure (10).

10. Implant according to one of claims 1 to 7 in conjunction with claim 5 or 6, **characterized in that** the reinforced zone (32) together with the radial reinforcing elements (34, 36) is made in one piece in the form of a reinforcing part, preferably weft-knitted or warp-knitted, and is attached to the separately produced basic structure (30).

11. Implant according to one of claims 1 to 10, **characterized by** non-resorbable material.

12. Implant according to claim 11 **characterized in that** the non-resorbable material comprises at least one of the substances selected from the following group: polypropylene, polyamide, polyester.

13. Implant according to one of claims 1 to 12, **characterized by** resorbable material.

14. Implant according to claim 13, **characterized in that** the resorbable material comprises at least one of the substances selected from the following group: copolymer made from lactide and glycolide, poly-p-dioxanone.

15. Implant according to claim 13 or 14, **characterized by** a stiffening element made from resorbable material.

16. Implant according to claim 15, **characterized in that** the stiffening element is formed as a coating of material of the implant.

17. Implant according to claim 15 or 16, **characterized in that** the stiffening element is formed as a film.
